# EUROPEAN PATENT APPLICATION

(11) **EP 4 745 116 A1**
(43) Date of publication of application: **20.05.2026**
(21) Application number: 24838886.0
(22) Date of filing: 12.07.2024
(51) Int. Cl.: C07C 41/24, A61P 35/00

(54) **PREPARATION METHOD FOR KRAS G12D INTERMEDIATE**

(30) Priority: 13.07.2023 CN 202310855917
(71) Applicant: Shanghai Senhui Medicine Co., Ltd., Shanghai 201203 (CN); Shanghai Shengdi Pharmaceutical Co., Ltd., Shanghai 201210 (CN); Jiangsu Hengrui Pharmaceuticals Co., Ltd., Lianyungang, Jiangsu 222047 (CN)
(72) Inventor: JIANG, Wei, Shanghai 201203 (CN); GAO, Junfeng, Shanghai 201203 (CN); ZHOU, Yinan, Shanghai 201203 (CN); JIANG, Jun, Shanghai 201203 (CN); HUANG, Jian, Shanghai 201203 (CN)
(74) Representative: Regimbeau
(86) International application number: PCT/CN2024/105153
(87) International publication number: WO 2025/011634

(57) **Abstract**

A preparation method for a KRAS G12D intermediate. Specifically, disclosed is a method for preparing a compound represented by formula (V-1). The preparation process is simple and can significantly reduce costs.

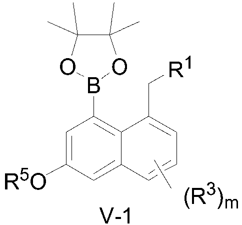

## Description

### TECHNICAL FIELD

The disclosure relates to a method for preparing KRAS G12D intermediates.

### BACKGROUND

RAS is one of the oncogenes with the highest mutation rate in tumors, and about 30% of human malignancies are associated with mutations of the RAS gene. The RAS family includes KRAS, NRAS, and HRAS, and KRAS mutations are the most common and account for approximately 85%. KRAS mutations are common in solid tumors, with highfrequency mutations in the three fatal cancers in humans: lung cancer (17%), colorectal cancer (33%), and pancreatic cancer (61%). A total of 97% of the KRAS gene mutations involve the mutation of amino acid residue 12 or 13, and G12D is an important mutation. Data analysis of Western populations shows that the G12D mutation is found in 36%, 12%, and 4% of pancreatic cancer, colorectal cancer, and non-small cell lung cancer patients, respectively.

After KRAS is activated, it regulates multiple functions such as cell proliferation, survival, migration, and metabolism through a plurality of downstream signaling pathways represented by RAF-MEK-ERK, PI3K-AKT-mTOR, and TIAM1-RAc. After the mutation of the KRAS gene, the protein is continuously activated, resulting in continuous activation of downstream signaling pathways, and thus promoting tumorigenesis. KRAS protein has long been considered an undruggable drug target as it lacks conventional small-molecule binding sites on its surface and it is extremely difficult to inhibit due to its ultrahigh affinity for guanylic acid. However, based on the importance and prevalence of abnormal KRAS activation in cancer progression, KRAS has been and remains a target of high interest for drug development. Currently, except for KRAS G12C inhibitors, there is a lack of KRAS inhibitors that are effective against other mutations, such that most patients with KRAS mutations remain non-medicated. G12D is a mutant widely and highly expressed in multiple tumors, and it is of important clinical significance to develop inhibitors against it.

Currently published related patent applications include WO2021041671A1, WO2020146613A1, WO2017172979A1, WO2020238791A1, WO2021000885A1, WO2022268051A1, etc.

The compounds represented by formula V-1 are important intermediates for the synthesis of KRAS G12D molecules. WO2021041671A discloses a synthesis method for similar intermediates; however, the method uses heavy metal catalysts in multiple steps, resulting in relatively high costs.

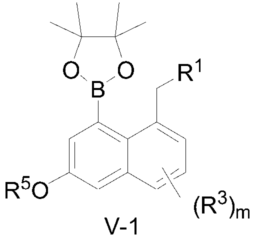

In view of cost reduction, the disclosure provides a new method for preparing KRAS G12D intermediates.

### SUMMARY

The disclosure provides a method for preparing a compound represented by formula II, comprising a step of preparing the compound represented by formula II from a compound represented by formula I: 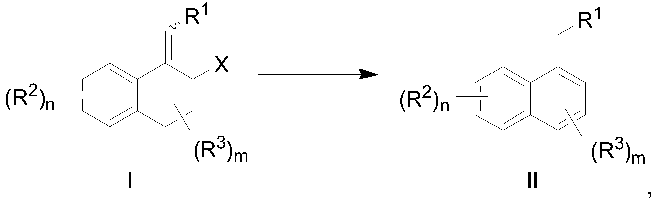 wherein:
X is a halogen;
R¹ is hydrogen or alkyl optionally substituted with one or more substituents Q;
R² are identical or different and are each independently selected from the group consisting of alkyl, alkoxy, halogen, hydroxy, sulfhydryl, oxo, -NRᵢRⱼ, cycloalkyl, heterocyclyl, aryl, and heteroaryl, wherein the alkyl, alkoxy, cycloalkyl, heterocyclyl, aryl, and heteroaryl are optionally substituted with one or more substituents Q;
R³ are identical or different and are each independently selected from the group consisting of alkyl, alkoxy, halogen, hydroxy, sulfhydryl, oxo, -NRᵢRⱼ, cycloalkyl, heterocyclyl, aryl, and heteroaryl, wherein the alkyl, alkoxy, cycloalkyl, heterocyclyl, aryl, and heteroaryl are optionally substituted with one or more substituents Q;
the substituents Q are each independently selected from the group consisting of C₁-C₆ alkyl, halogen, hydroxy, sulfhydryl, -NRᵢRⱼ, oxo, thio, -C(O)Rₖ, -C(O)ORₖ, -S(O)Rₖ,-S(O)ORₖ, -S(O)(O)Rₖ, -S(O)(O)ORₖ, -C(S)Rₖ, nitro, cyano, C₁-C₆ alkoxy, C₁-C₆ alkylthioether group, C₂-C₆ alkenyl, C₂-C₆ alkynyl, 3- to 10-membered cycloalkyl, 3- to 10-membered heterocyclyl, 6- to 10-membered aryl, 5- to 10-membered heteroaryl, 8- to 12-membered fused cycloaryl, and 5- to 12-membered fused heteroaryl;
Rᵢ and Rⱼ are each independently selected from the group consisting of hydrogen, hydroxy, C₁-C₆ alkyl, and C₁-C₆ alkoxy; Rₖ is independently selected from the group consisting of hydrogen, C₁-C₆ alkyl, C₁-C₆ haloalkyl, C₁-C₆ alkoxy, hydroxy, and -NRᵢRⱼ, wherein the alkyl and alkoxy are optionally substituted with one or more substituents selected from the group consisting of C₁-C₆ alkyl, halogen, hydroxy, sulfhydryl, -NRᵢRⱼ, oxo, thio, carboxyl, nitro, cyano, C₁-C₆ alkoxy, C₁-C₆ alkylthioether group, C₂-C₆ alkenyl, C₂-C₆ alkynyl, 3- to 10-membered cycloalkyl, 3- to 10-membered heterocyclyl, 6- to 10-membered aryl, and 5- to 10-membered heteroaryl;
n is 0, 1, 2, 3, or 4;
m is 0, 1 or 2, or 3.

In some embodiments, R¹ is hydrogen or C₁-C₆ alkyl optionally substituted with one or more substituents Q.

In some embodiments, R² and R³ are each independently selected from the group consisting of C₁-C₆ alkyl, C₁-C₆ alkoxy, halogen, hydroxy, sulfhydryl, oxo, -NRᵢRⱼ, 3- to 10-membered cycloalkyl, and 3- to 10-membered heterocyclyl, wherein the alkyl, alkoxy, cycloalkyl, and heterocyclyl are optionally substituted with one or more substituents Q. In some embodiments, R² and R³ are each independently selected from the group consisting of C₁-C₆ alkyl, C₁-C₆ alkoxy, halogen, hydroxy, oxo, amino, 3- to 10-membered cycloalkyl, and 3- to 10-membered heterocyclyl, wherein the alkyl, alkoxy, cycloalkyl, and heterocyclyl are optionally substituted with one or more substituents selected from the group consisting of C₁-C₆ alkyl, C₁-C₆ alkoxy, halogen, hydroxy, amino, carboxyl, nitro, and cyano.

In some embodiments, R² and R³ are each independently selected from the group consisting of C₁-C₆ alkyl, C₁-C₆ alkoxy, halogen, oxo, hydroxy, and amino, wherein the alkyl and alkoxy are optionally substituted with one or more substituents selected from the group consisting of C₁-C₆ alkyl, C₁-C₆ alkoxy, halogen, hydroxy, amino, carboxyl, nitro, and cyano.

In some embodiments, R² and R³ are each independently selected from the group consisting of C₁-C₆ alkyl, C₁-C₆ alkoxy, halogen, and hydroxy.

In some embodiments, R² are each independently selected from the group consisting of halogen and C₁-C₆ alkoxy, and n is 2.

In some embodiments, the substituents Q are each independently selected from the group consisting of C₁-C₆ alkyl, halogen, hydroxy, sulfhydryl, -NRᵢRⱼ, oxo, thio, -C(O)Rₖ,-C(O)ORₖ, -S(O)Rₖ, -S(O)ORₖ, -S(O)(O)Rₖ, -S(O)(O)ORₖ, -C(S)Rₖ, nitro, cyano, C₁-C₆ alkoxy, C₁-C₆ alkylthioether group, C₂-C₆ alkenyl, and C₂-C₆ alkynyl.

In some embodiments, the substituents Q are each independently selected from the group consisting of C₁-C₆ alkyl, halogen, hydroxy, NRᵢRⱼ, oxo, -C(O)Rₖ, -C(O)ORₖ, cyano, C₁-C₆ alkoxy, C₂-C₆ alkenyl, and C₂-C₆ alkynyl.

In some embodiments, the aforementioned method for preparing a compound represented by formula II comprises a step of preparing a compound represented by formula II-1 from a compound represented by formula I-1: 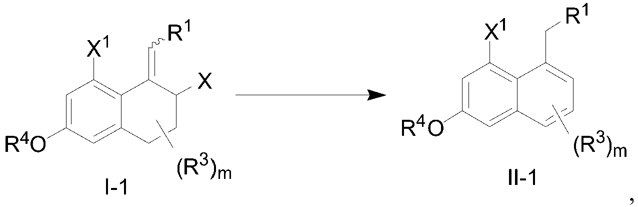 wherein:
X¹ is a halogen;
R⁴ is C₁-C₆ alkyl optionally substituted with one or more substituents Q;
R¹, R³, X, Q, and m are as defined in general formula I.

The disclosure further provides a method for preparing a compound represented by formula III-1, comprising the aforementioned step of preparing the compound represented by formula II-1 from the compound represented by formula I-1, and further comprising a step of hydroxylating the compound represented by formula II-1 to give the compound represented by formula III-1: 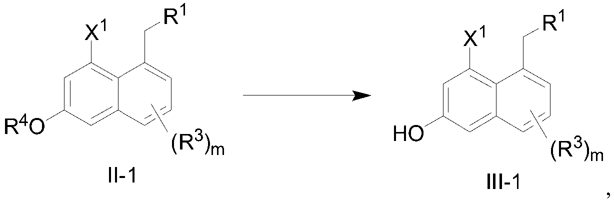 wherein R¹, R³, R⁴, X¹, and m are as defined in general formula I-1.

The disclosure further provides a method for preparing a compound represented by formula IV-1, comprising the aforementioned step of hydroxylating the compound represented by formula II-1 to give the compound represented by formula III-1, and further comprising a step of preparing the compound represented by formula IV-1 from the compound represented by formula III-1: 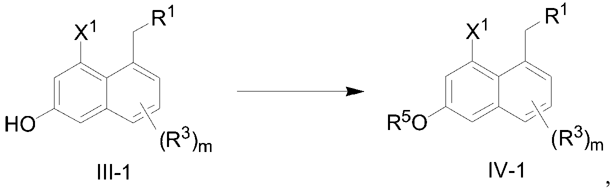
wherein R¹, R³, X¹, and m are as defined in general formula I-1;
R⁵ is a hydroxy protecting group selected from the group consisting of benzyl, benzoyl, tert-butyryl, trimethylsilyl, tert-butyldiphenylsilyl, and methoxymethyl.

The disclosure further provides a method for preparing a compound represented by formula V-1, comprising the aforementioned step of preparing the compound represented by formula IV-1 from the compound represented by formula III-1, and further comprising a step of preparing the compound represented by formula V-1 from the compound represented by formula IV-1: 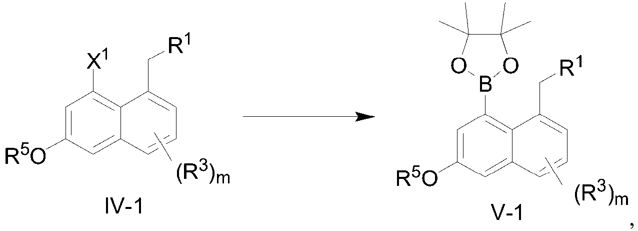 wherein R¹, R³, R⁵, X¹, and m are as defined in general formula IV-1.

The disclosure further provides a compound represented by formula f:

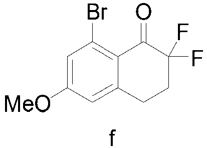

The disclosure further provides a compound represented by formula I-2:

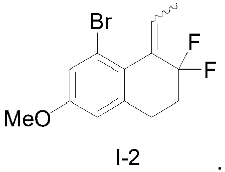

The disclosure further provides a compound represented by formula II-2:

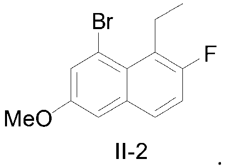

The disclosure further provides a compound represented by formula III-2:

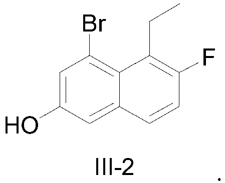

The disclosure further provides a compound represented by formula IV-2:

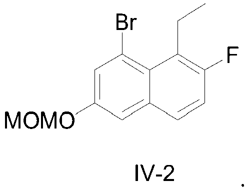

The disclosure further provides a method for preparing a compound represented by formula V-2, comprising the following steps:

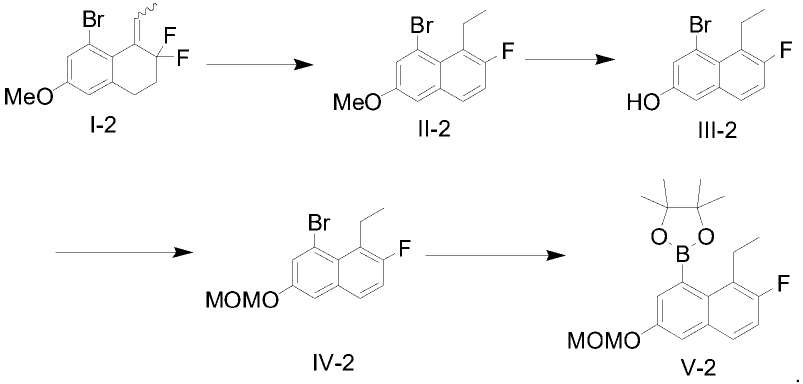

In some embodiments, the method for preparing a compound represented by formula V-2 comprises the following specific steps:

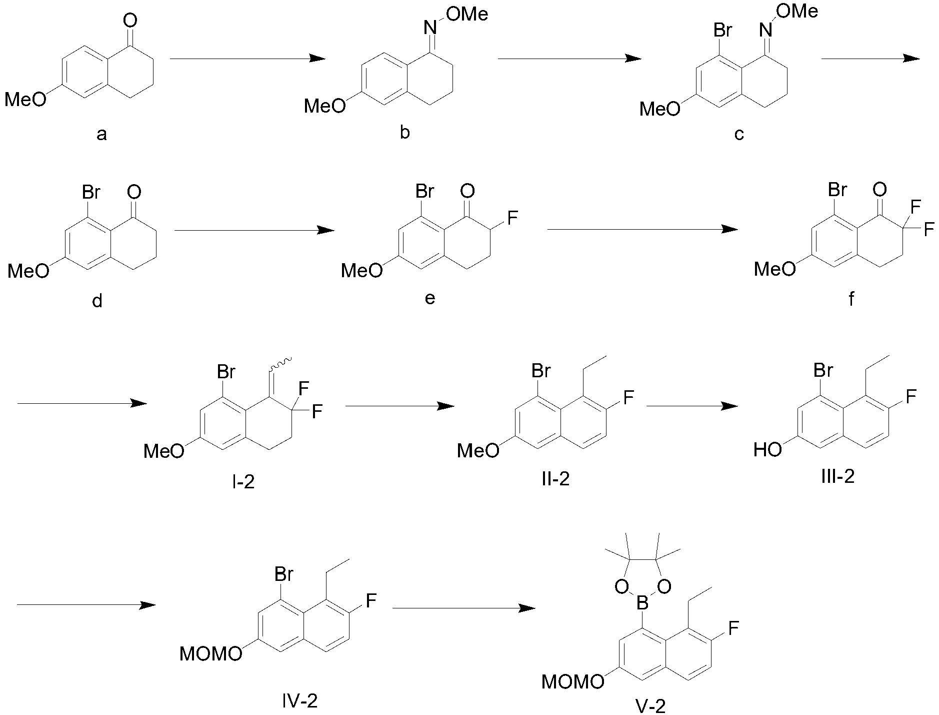

The disclosure further provides a method for preparing a compound represented by formula V-3, comprising the following steps:

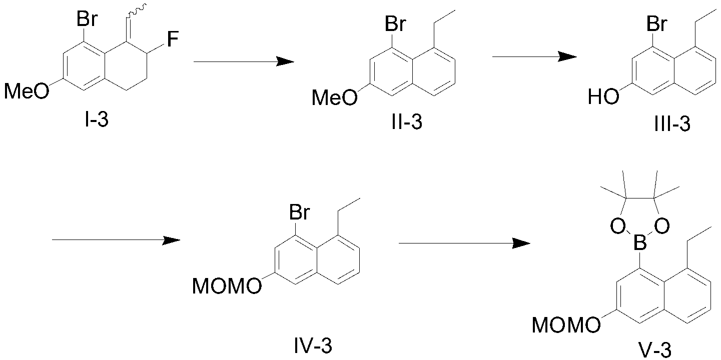

The disclosure further provides a method for preparing a KRAS G12D compound, comprising the aforementioned step of preparing the compound represented by formula II from the compound represented by formula I.

The compound represented by formula II in the disclosure is used for preparing a KRAS G12D compound. In some embodiments, the compound represented by formula II is prepared by the method provided in the disclosure.

The disclosure further provides a method for preparing a compound represented by formula A, comprising the aforementioned steps of preparing the compound represented by formula V-2, and further comprises the following steps:

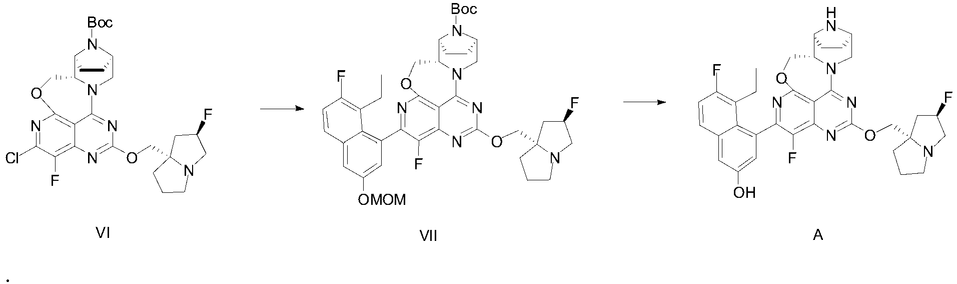

WO2022268051A discloses a preparation method for the compound represented by formula A and is incorporated herein by reference in its entirety.

Another aspect of the disclosure relates to a pharmaceutical composition comprising a therapeutically effective amount of a compound of general formula (I) prepared by the aforementioned method, or a tautomer, mesomer, racemate, enantiomer, or diastereomer thereof, or a mixture thereof, or a pharmaceutically acceptable salt thereof, and one or more pharmaceutically acceptable carriers, diluents, or excipients.

The "amino protecting group" described in the disclosure is a suitable amino protecting group known in the art. See amino protecting groups in the literature ("Protective Groups in Organic Synthesis", 5th. Ed. T. W. Greene & P. G. M. Wuts). By way of example, the amino protecting group is selected from the group consisting of tert-butoxycarbonyl, acetyl, benzyl, allyl, and p-methoxybenzyl, or the amino protecting group, together with the nitrogen atom to which it is attached, forms a lactam, such as phthaloyl. For the method of removing the protecting group, reference can also be made to the method of removing an amino protecting group in the literature ("Protective Groups in Organic Synthesis", 5th. Ed. T. W. Greene & P. G. M. Wuts), and the relevant contents are incorporated into the specification. By way of example, benzyl is removed by hydrogen/a metal catalyst, wherein the metal catalyst is selected from the group consisting of, but is not limited to, at least one of palladium on carbon, palladium hydroxide, platinum oxide, palladium, palladium on aluminum oxide, platinum on activated carbon, and Raney nickel, and is preferably palladium on carbon or palladium hydroxide; tert-butyloxycarbonyl (Boc) is removed under acidic conditions, and a reagent providing the acidic conditions is preferably selected from the group consisting of methanesulfonic acid, hydrochloric acid, and trifluoroacetic acid.

The hydride source described in the disclosure is well known or determinable by those skilled in the art and is selected from the group consisting of, but is not limited to, at least one of lithium aluminum hydride, lithium triethylborohydride, diisobutyl aluminum hydride, sodium borohydride, sodium bis(2-methoxyethoxy)aluminium hydride, and hydrogen/a metal catalyst. Further, the metal catalyst is selected from the group consisting of, but is not limited to, at least one of palladium on carbon, palladium hydroxide, platinum oxide, palladium, palladium on aluminum oxide, platinum on activated carbon, and Raney nickel, and is preferably palladium on carbon or palladium hydroxide.

The salt of the compound represented by formula (I-B) described in the disclosure is a salt formed by a reaction of the compound represented by formula (I-B) with an acid. The acid used may be hydrochloric acid, maleic acid, sulfuric acid, phosphoric acid, or the like, preferably hydrochloric acid.

Reagents that provide the alkaline conditions in the disclosure include organic alkalis and inorganic alkalis. The organic alkalis include, but are not limited to, triethylamine, *N,N-*diisopropylethylamine, n-butyllithium, lithium diisopropylamide, lithium bis(trimethylsilyl)amide, potassium acetate, sodium tert-butoxide, potassium tert-butoxide, and sodium n-butoxide, and the inorganic alkalis include, but are not limited to, sodium, potassium, sodium hydride, potassium phosphate, sodium carbonate, potassium carbonate, potassium acetate, cesium carbonate, sodium hydroxide, and lithium hydroxide.

Reagents that provide the acidic conditions in the disclosure include, but are not limited to, hydrogen chloride, hydrogen chloride in 1,4-dioxane, trifluoroacetic acid, formic acid, acetic acid, hydrochloric acid, sulfuric acid, methanesulfonic acid, nitric acid, phosphoric acid, p-toluenesulfonic acid, Me₃SiCl, and TMSOTf.

The above reactions are preferably conducted in solvents. The solvents used include, but are not limited to, acetic acid, methanol, ethanol, n-butanol, toluene, tetrahydrofuran, dichloromethane, petroleum ether, ethyl acetate, n-hexane, dimethyl sulfoxide, 1,4-dioxane, water, or N,N-dimethylformamide.

### Detailed Description of the Invention

Unless otherwise stated, the terms used in the specification and claims have the following meanings.

The term "alkyl" refers to a saturated aliphatic hydrocarbon group, including linear and branched groups of 1 to 20 carbon atoms, and alkyl groups containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, various branched isomers thereof, etc. Alkyl may be substituted or unsubstituted, and when it is substituted, the substituent may be substituted at any available point of attachment, preferably with one or more groups independently selected from the group consisting of halogen, hydroxy, oxo, cyano, amino, C₁₋₆ alkyl, C₁₋₆ alkoxy, 3- to 6-membered cycloalkyl, and 3- to 6-membered heterocycloalkyl, wherein the alkyl, alkoxy, cycloalkyl, or heterocycloalkyl is optionally substituted with halogen, hydroxy, nitro, cyano, or amino.

The term "alkenyl" refers to an unsaturated aliphatic linear or branched hydrocarbon group containing one or more carbon-carbon double bonds. Exemplary alkenyl groups include C2-C8, C2-C7, C2-C6, C2-C4, C3-C12, and C3-C6 alkenyl groups, including but not limited to ethenyl (i.e., vinyl), 1-propenyl, 2-propenyl (i.e., allyl), 2-methyl-1-propenyl, 1-butenyl, 2-butenyl (i.e., crotyl), etc. Alkenyl used in any context herein is optionally substituted in the same manner as alkyl.

The term "alkynyl" refers to an unsaturated aliphatic linear or branched hydrocarbon group containing one or more carbon-carbon triple bonds. Exemplary alkynyl groups include C2-C8, C2-C7, C2-C6, C2-C4, C3-C12, and C3-C6 alkynyl groups, including but not limited to ethynyl, prop-1-ynyl, but-1-ynyl, pent-1-ynyl, pent-4-ynyl, and pent-1,4-diynyl. Alkynyl used in any context herein is optionally substituted in the same manner as alkyl.

The term "cycloalkyl" refers to a saturated or partially unsaturated, monocyclic or polycyclic hydrocarbon substituent, and the cycloalkyl ring contains 3 to 20 carbon atoms, preferably 3 to 6 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, etc. Polycyclic cycloalkyl groups include spiro-ring, fused-ring, and bridged-ring cycloalkyl groups. Cycloalkyl may be substituted or unsubstituted, and when it is substituted, the substituent may be substituted at any available point of attachment, preferably with one or more groups independently selected from the group consisting of halogen, hydroxy, oxo, cyano, amino, C₁₋₆ alkyl, C₁₋₆ alkoxy, 3- to 6-membered cycloalkyl, and 3- to 6-membered heterocycloalkyl, wherein the alkyl, alkoxy, cycloalkyl, or heterocycloalkyl is optionally substituted with halogen, hydroxy, nitro, cyano, or amino.

The term "heterocycloalkyl" refers to a saturated or partially unsaturated, monocyclic or polycyclic hydrocarbon substituent containing 3 to 20 ring atoms, wherein one or more of the ring atoms are heteroatoms selected from the group consisting of nitrogen, oxygen, and S(O)ₘ (where m is an integer from 0 to 2), but a ring moiety of -O-O-, -O-S-, or -S-S- is excluded, and the other ring atoms are carbon atoms. Preferably, it contains 3 to 12 ring atoms, 1-4 of which are heteroatoms; more preferably, it contains 3 to 7 ring atoms. Non-limiting examples of "heterocycloalkyl" groups include: 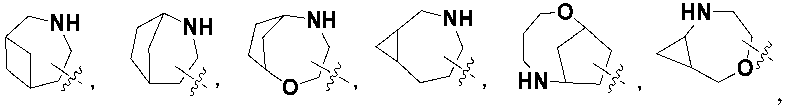 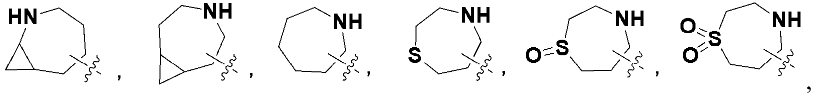 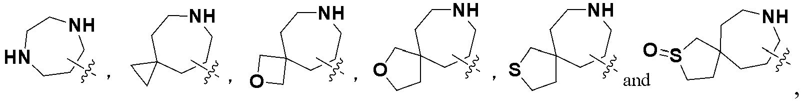 etc.

The heterocycloalkyl ring may be fused to an aryl or heteroaryl ring, wherein the ring attached to the parent structure is heterocycloalkyl; its non-limiting examples include: 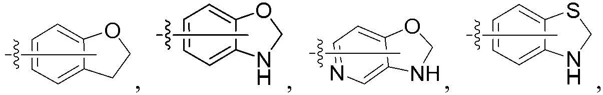 etc.

Heterocycloalkyl may be optionally substituted or unsubstituted, and when it is substituted, the substituent is preferably one or more groups independently selected from the group consisting of halogen, hydroxy, oxo, cyano, amino, C₁₋₆ alkyl, C₁₋₆ alkoxy, 3-to 6-membered cycloalkyl, and 3- to 6-membered heterocycloalkyl, wherein the alkyl, alkoxy, cycloalkyl, or heterocycloalkyl is optionally substituted with halogen, hydroxy, nitro, cyano, or amino.

The term "cycloalkenyl" refers to an unsaturated monocyclic or polycyclic hydrocarbon substituent containing one or more carbon-carbon double bonds. Cycloalkenyl groups contain 3 to 20 carbon atoms and include C2-C8, C4-C6, C8-C12, C14-C18, and C16-C20 cycloalkenyl groups. In addition, cycloalkenyl may be fused to aryl or heteroaryl. Exemplary cycloalkenyl groups include, but are not limited to:

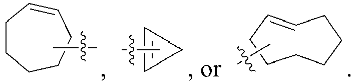

In addition, cycloalkenyl may be substituted or unsubstituted, and when it is substituted, the substituent may be substituted at any available point of attachment, preferably with one or more groups independently selected from the group consisting of halogen, C₁₋₃ alkyl, and C₁₋₃ alkoxy. (Adjust as needed!)

The term "cycloalkynyl" refers to an unsaturated monocyclic or polycyclic hydrocarbon substituent containing one or more carbon-carbon triple bonds. Cycloalkynyl groups contain 3 to 20 carbon atoms and include C2-C8, C4-C6, C8-C12, C14-C18, and C16-C20 cycloalkynyl groups. In addition, cycloalkynyl may be fused to aryl or heteroaryl. Exemplary cycloalkynyl groups include, but are not limited to:

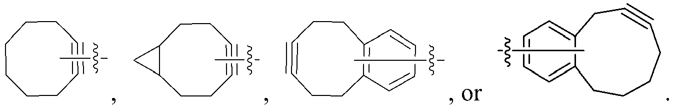

In addition, cycloalkynyl may be substituted or unsubstituted, and when it is substituted, the substituent may be substituted at any available point of attachment, preferably with one or more groups independently selected from the group consisting of halogen, C₁₋₃ alkyl, and C₁₋₃ alkoxy.

The term "heterocycloalkenyl" refers to an unsaturated monocyclic or polycyclic hydrocarbon substituent containing 3 to 20 ring atoms, wherein one or more of the ring atoms are heteroatoms selected from the group consisting of nitrogen, oxygen, and S(O)ₘ (where m is an integer from 0 to 2), but a ring moiety of -O-O-, -O-S-, or -S-S- is excluded; the other ring atoms are carbon atoms, and one or more carbon-carbon double bonds are contained. Heterocycloalkenyl groups contain 3 to 20 carbon atoms and preferably include C2-C8, C4-C6, C8-C12, C14-C18, and C16-C20 heterocycloalkenyl groups. In addition, heterocycloalkenyl may be fused to aryl or heteroaryl. Exemplary heterocyclyl groups include, but are not limited to: 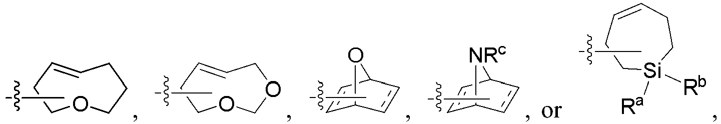 wherein R^{a} and
R^{b} are each independently selected from the group consisting of hydrogen, C₁₋₆ alkyl, and aryl; R^{c} is selected from the group consisting of hydrogen and C₁₋₆ alkyl. In addition, heterocycloalkenyl may be substituted or unsubstituted, and when it is substituted, the substituent may be substituted at any available point of attachment, preferably with one or more groups independently selected from the group consisting of halogen, C₁₋₃ alkyl, and C₁₋₃ alkoxy.

The term "heterocycloalkynyl" refers to an unsaturated monocyclic or polycyclic hydrocarbon substituent containing 3 to 20 ring atoms, wherein one or more of the ring atoms are heteroatoms selected from the group consisting of nitrogen, oxygen, and S(O)ₘ (where m is an integer from 0 to 2),
but a ring moiety of -O-O-, -O-S-, or -S-S- is excluded; the other ring atoms are carbon atoms, and one or more carbon-carbon triple bonds are contained. Heterocycloalkynyl groups contain 3 to 20 carbon atoms and include C2-C8, C4-C6, C8-C12, C14-C18, and C16-C20 heterocycloalkynyl groups. In addition, heterocycloalkynyl may be fused to aryl or heteroaryl. Exemplary heterocycloalkynyl groups include, but are not limited to: 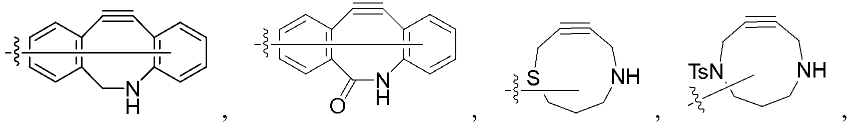 or

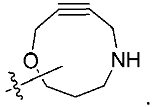

In addition, heterocycloalkynyl may be substituted or unsubstituted, and when it is substituted, the substituent may be substituted at any available point of attachment, preferably with one or more groups independently selected from the group consisting of halogen, C₁₋₃ alkyl, and C₁₋₃ alkoxy.

The term "alkoxy" refers to -O-(alkyl), wherein the alkyl is as defined above. Non-limiting examples of alkoxy groups include methoxy, ethoxy, propoxy, and butoxy. Alkoxy may be optionally substituted or unsubstituted, and when it is substituted, the substituent is preferably one or more groups independently selected from the group consisting of halogen, hydroxy, oxo, cyano, amino, C₁₋₆ alkyl, C₁₋₆ alkoxy, 3- to 7-membered cycloalkyl, and 3- to 7-membered heterocycloalkyl, wherein the alkyl, alkoxy, cycloalkyl, or heterocycloalkyl is optionally substituted with halogen, hydroxy, nitro, cyano, or amino.

Likewise, the definitions of "cycloalkoxy" and "heterocycloalkoxy" are similar to the above definition of "alkoxy".

The term "alkylthio" refers to -S-(alkyl), wherein the alkyl is as defined above. Non-limiting examples of alkoxy groups include: methylthio, ethylthio, propylthio, and butylthio. Alkylthio may be optionally substituted or unsubstituted, and when it is substituted, the substituent is preferably one or more groups independently selected from the group consisting of C₁₋₆ alkoxy, 3- to 6-membered cycloalkyl, 3- to 6-membered heterocycloalkyl, 3- to 6-membered cycloalkoxy, 3- to 6-membered heterocycloalkoxy, C₁₋₆ alkylthio, 3- to 6-membered cycloalkylthio, and 3- to 6-membered heterocycloalkylthio, wherein the alkoxy, cycloalkyl, heterocycloalkyl, cycloalkoxy, heterocycloxy, alkylthio, cycloalkylthio, and heterocycloalkylthio are optionally substituted with halogen, hydroxy, cyano, or amino.

Likewise, the definitions of "cycloalkylthio" and "heterocycloalkylthio" are similar to the above definition of "alkylthio".

"Monovalent group" refers to a group formed by "formally" eliminating a monovalent atom or group from a compound. "-ylene" refers to a group formed by "formally" eliminating two monovalent atoms or groups of atoms or one divalent atom or group of atoms from a compound.

The term "alkylene" refers to the remainder of an alkane molecule after 2 hydrogen atoms are removed from the alkane molecule, including linear and branched -ylene groups of 1 to 20 carbon atoms. Non-limiting examples of alkylene groups containing 1 to 6 carbon atoms include methylene (-CH₂-) and ethylene (e.g., -CH₂CH₂- or -CH(CH₃)-). Unless otherwise specified, alkylene may be substituted or unsubstituted, and when it is substituted, the substituent may be substituted at any available point of attachment, preferably with one or more groups independently selected from the group consisting of halogen, hydroxy, cyano, amino, C₁₋₆ alkyl, and C₁₋₆ alkoxy. (Adjust as needed!)

Likewise, the definitions of "alkyleneoxy", "alkenylene", "alkenyleneoxy", "cycloalkylene", and "heterocycloalkylene" are similar to that of "alkylene".

The term "aryl" refers to a 6- to 14-membered, preferably 6- to 12-membered, all-carbon monocyclic or fused polycyclic (i.e., rings sharing a pair of adjacent carbon atoms) group having a conjugated π-electron system, such as phenyl and naphthyl. The aryl ring may be fused to a heteroaryl, heterocycloalkyl, or cycloalkyl ring, wherein the ring attached to the parent structure is the aryl ring; its non-limiting examples include:

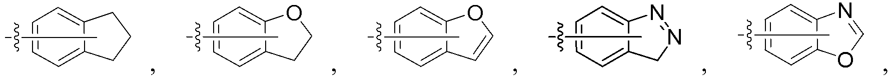

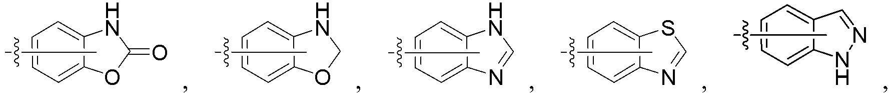

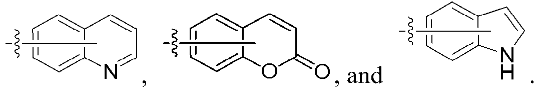

Aryl may be substituted or unsubstituted, and when it is substituted, the substituent is preferably one or more groups independently selected from the group consisting of halogen, hydroxy, oxo, nitro, cyano, C₁₋₆ alkyl, C₁₋₆ alkoxy, C₂₋₆ alkenyloxy, C₂₋₆ alkynyloxy, 3- to 6-membered cycloalkoxy, 3- to 6-membered heterocycloalkoxy, C₃₋₈ cycloalkenyloxy, and 5- to 6-membered aryl or heteroaryl, wherein the C₁₋₆ alkyl, C₁₋₆ alkoxy, C₂₋₆ alkenyloxy, C₂₋₆ alkynyloxy, 3- to 6-membered cycloalkoxy, 3- to 6-membered heterocycloalkoxy, 3- to 8-membered cycloalkenyloxy, and 5- to 6-membered aryl or heteroaryl are optionally substituted with one or more groups selected from the group consisting of halogen, hydroxy, cyano, amino, C₁₋₆ alkyl, and C₁₋₆ alkoxy.

The term "heteroaryl" refers to a heteroaromatic system containing 1 to 4 heteroatoms and 5 to 14 ring atoms, wherein the heteroatoms are selected from the group consisting of oxygen, sulfur, and nitrogen. Preferably, heteroaryl is 6- to 12-membered. More preferably, heteroaryl is 5- or 6-membered. For example, its non-limiting examples include imidazolyl, furanyl, thienyl, thiazolyl, pyrazolyl, oxazolyl, isoxazolyl, pyrrolyl, tetrazolyl, pyridyl, pyrimidinyl, thiadiazole, pyrazinyl, triazolyl, indazolyl, benzimidazolyl, 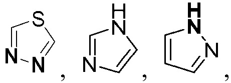 etc.

The heteroaryl ring may be fused to an aryl, heterocycloalkyl, or cycloalkyl ring, wherein the ring attached to the parent structure is the heteroaryl ring; its non-limiting examples include:

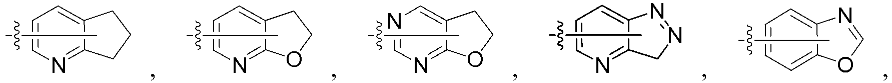

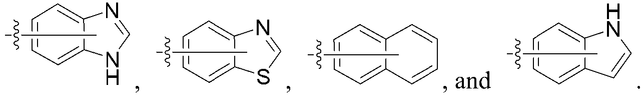

Heteroaryl may be optionally substituted or unsubstituted, and when it is substituted, the substituent is preferably one or more groups independently selected from the group consisting of halogen, hydroxy, cyano, amino, C₁₋₆ alkyl, and C₁₋₆ alkoxy (Adjustments are needed here according to the claims!).

The term "spiro" refers to compounds in which two rings share one atom. Non-limiting examples of spirocycloalkyl groups include:

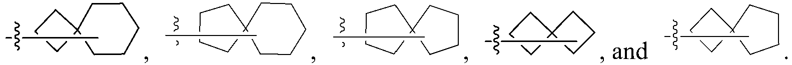

The term "fused" refers to compounds formed by fusing two or more rings by sharing two adjacent atoms. Non-limiting examples of fused cycloalkyl groups include:

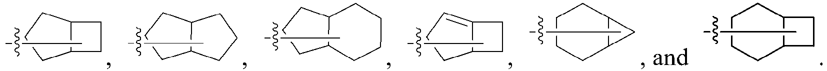

The term "bridged" refers to a structure formed by two or more cyclic structures sharing two non-adjacent ring atoms. According to the number of constituent rings, it may be bicyclic, tricyclic, tetracyclic, or polycyclic bridged cycloalkyl, preferably bicyclic, tricyclic, or tetracyclic, and more preferably bicyclic or tricyclic. Non-limiting examples of bridged cycloalkyl groups include:

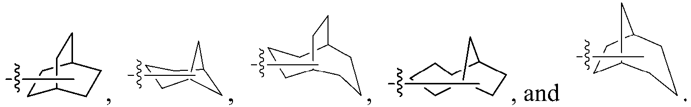

The term "heterocycle" means that the atoms that comprise the ring include not only carbon atoms but also other atoms; this term encompasses heterocycloalkyl and heteroaromatic rings.

The term "hydroxy" refers to the -OH group.

The term "halogen" refers to fluorine, chlorine, bromine, or iodine.

The term "cyano" refers to -CN.

The term "amino" refers to -NH₂.

The term "nitro" refers to -NO₂.

The term "oxo" refers to the =O substituent.

"Substituted" means that one or more, preferably up to 5, and more preferably 1 to 3, hydrogen atoms in the group are independently substituted with a corresponding number of substituents. When the substituent is a ketone or oxo (i.e., =O), two (2) hydrogens on the atom are replaced.

The purity or content described in the disclosure is determined by HPLC analysis, and compound characterization data are obtained by analysis of nuclear magnetic resonance spectra; the reagents used in the disclosure are commercially available.

### DETAILED DESCRIPTION

The disclosure is explained below in more detail with reference to examples. The examples of the disclosure are only used to illustrate the technical solutions of the disclosure, and the essence and scope of the disclosure are not limited to these examples.

### Example 1: Preparation of 6-Methoxy-3,4-dihydronaphthalen-1(2H)-one-methyl oxime

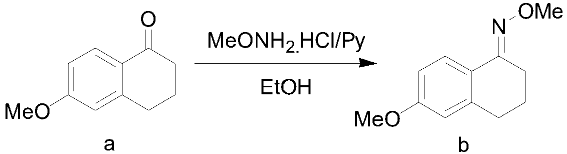

Compound a (100 g, 1.0 eq.) and ethanol (1000 mL, 10 V) were added to a 2 L reaction flask and stirred at 15-25 °C to dissolve the compound. Methoxyamine hydrochloride (95 g, 2.0 eq) and pyridine (108 g, 2.4 eq) were added. After the addition, the mixture was stirred at 15-25 °C for 2-3 h. HPLC analysis showed that the reaction was complete. The reaction mixture was concentrated under reduced pressure to remove ethanol, and ethyl acetate (500 mL, 5 V) was added to dissolve the residue. The solution was washed once with 2 M HCl (200 mL, 2 V), washed once with 5% sodium bicarbonate (200 mL, 2 V), washed once with saturated brine (200 mL, 2 V), dried over anhydrous sodium sulfate, filtered, and concentrated under reduced pressure to dryness to give a crude product (120 g, yield: 104%).

¹H NMR (400 MHz, CDCl3) δ 7.91-7.94 (d, 1H), 6.75-6.78 (dd, 2.7 Hz, 1H), 6.64-6.65 (d, 1H), 3.96 (s, 3H), 3.81 (s, 3H), 2.70-2.74 (t, 4H), 1.81-1.87 (m, 2H).

LCMS (ESI): m/z 206[M+H]

### Example 2: Preparation of 8-Bromo-6-methoxy-3,4-dihydronaphthalen-1(2H)-one-methyl oxime

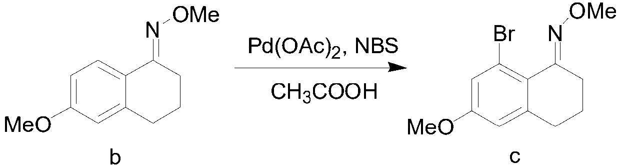

Compound b (96.7 g, 1.0 eq) and acetic acid (967 mL, 10 V) were added to a reaction flask and stirred to dissolve the compound. In a nitrogen atmosphere, palladium acetate (3.17 g, 0.03 eq) and NBS (84.8 g, 1.0 eq) were added. The mixture was heated to 50-60 °C and incubated for 1 h. HPLC analysis showed that the reaction was complete. The reaction mixture was cooled and concentrated under reduced pressure to remove acetic acid. Ethyl acetate (967 mL, 10 V) and H₂O (483.5 mL, 5 V) were added, and layers formed. The organic layer was washed once with 5% sodium bicarbonate (483.5 mL, 5 V), washed once with saturated sodium chloride (483.5 mL, 5 V), dried over anhydrous sodium sulfate, filtered, and concentrated under reduced pressure to dryness to give a crude product (145 g, yield: 105%).

¹H NMR (400 MHz, CDCl3) δ 7.07-7.08 (d, 1H), 6.64-6.65 (d, 1H), 4.01 (s, 3H), 3.79 (s, 3H), 2.72-2.75 (t, 2H), 2.57-2.50 (t, 2H), 1.79-1.67 (m, 2H)

LCMS (ESI): m/z 284[M+H], 286[M+3H]

### Example 3: Preparation of 8-Bromo-6-methoxy-3,4-dihydronaphthalen-1(2H)-one

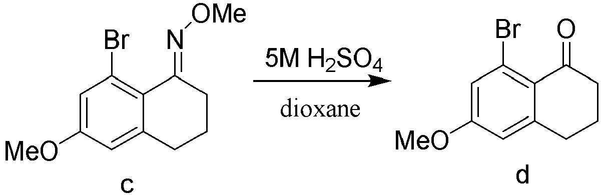

Compound c (46 g, 1.0 eq) and 1,4-dioxane (184 mL, 4 V) were added to a reaction flask. In a nitrogen atmosphere, 5 M sulfuric acid (552 mL, 12 V) was added. The mixture was heated to 40-50 °C and left to react at that temperature for 2-3 h. HPLC analysis showed that the reaction was complete. The reaction mixture was cooled. With the internal temperature controlled at 10-20 °C, a 12 M aqueous NaOH solution (a 460 mL solution prepared using 220 g of NaOH) was added dropwise to adjust the pH to 6-7. Then, ethyl acetate (460 mL, 10 V) was added for extraction, and layers formed. The organic layer was washed once with saturated sodium chloride (200 mL, 4.3 V), dried over anhydrous sodium sulfate, filtered, and concentrated to dryness to give a crude product (43 g). Ethyl acetate (46 mL, 1 V) was added to the crude product, and the mixture was heated at reflux until the crude product was completely dissolved. Petroleum ether (300 mL, 6.5 V) was added, and the mixture was incubated for 30 min and filtered. The filtrate was heated at reflux, and petroleum ether (130 mL, 2.8 V) was added to the filtrate. The mixture was cooled to 0-5 °C, stirred at that temperature, and filtered. The filter cake was dried *in vacuo* to give the title compound (30 g, yield: 72%).

¹H NMR (400 MHz, CDCl3) δ 7.10-7.11 (d, 1H), 6.69-6.70 (d, 1H), 3.84 (s, 3H), 2.92-2.95 (t, 2H), 2.63-2.67 (t, 2H), 2.04-2.10 (m, 2H).

LCMS (ESI): m/z 255[M+H], 257[M+3H]

### Example 4: Preparation of 8-Bromo-2-fluoro-6-methoxy-3,4-dihydronaphthalen-1(2H)-one

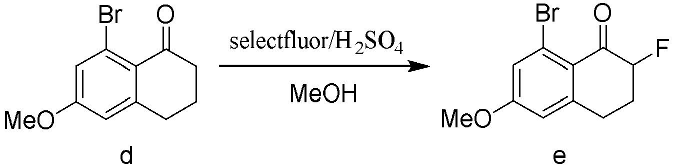

Compound d (30 g, 1.0 eq) and methanol (300 mL, 10 V) were added to a reaction flask and stirred to dissolve the compound. In a nitrogen atmosphere, selectfluor (48 g, 1.15 eq, manufacturer: Leyan, lot No. Lf0905165866) and concentrated sulfuric acid (0.6 mL, 0.02 V) were added. The mixture was heated to 50-60 °C and left to react at that temperature for 3 h. HPLC analysis showed that the reaction was complete. The reaction mixture was cooled and concentrated under reduced pressure to remove methanol. Then, dichloromethane (450 mL, 15 V) and H₂O (450 mL, 15 V) were added for extraction, and layers formed. The organic phase was washed once with a 5% aqueous sodium bicarbonate solution (200 mL, 6.67 V), washed once with saturated sodium chloride (200 mL, 6.67 V), dried over anhydrous sodium sulfate, filtered, and concentrated under reduced pressure to dryness to give a crude product (31.4 g, yield: 97%).

¹H NMR (400 MHz, CDCl3) δ 7.12-7.13 (d, 1H), 6.68-6.69 (d, 1H), 4.96-5.13 (m, 1H), 3.85 (d, 3H), 3.11-3.17 (m, 2H), 2.28-2.53 (m, 2H)

LCMS (ESI): m/z 274[M+H], 276[M+3H]

### Example 5: Preparation of 8-Bromo-2,2-difluoro-6-methoxy-3,4-dihydronaphthalen-1(2H)-one

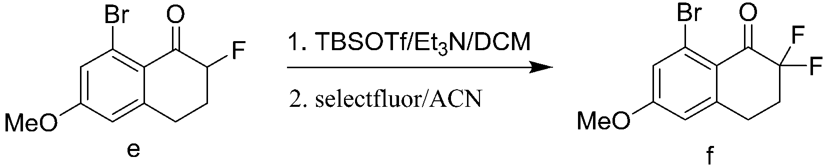

Compound e (30 g, 1.0 eq), DCM (300 mL, 10 V), and triethylamine (55.5 g, 5.0 eq) were added to a reaction flask and stirred to dissolve the compounds. In a nitrogen atmosphere, with the temperature controlled at 15-25 °C, TBSOTf (58 g, 2.0 eq) was added dropwise. After the addition, the mixture was stirred at 15-25 °C for 1-2 h. HPLC analysis showed that the reaction was complete. The reaction mixture was concentrated under reduced pressure to remove DCM. In a nitrogen atmosphere, ACN (240 mL, 8 V) and selectfluor (42.8 g, 1.1 eq) were added. The mixture was stirred at 15-25 °C for 1-2 h. HPLC analysis showed that the reaction was complete. With the temperature controlled at 15-25 °C, water (240 mL, 8 V) was slowly added dropwise to the reaction system. After the addition, the mixture was stirred at 15-25 °C for 1 h and filtered. The filter cake was rinsed with a small amount of water and dried *in vacuo* to give the title compound (26 g, yield: 81%). ¹H NMR (400 MHz, CDCl3) δ 7.19-7.20 (d, 1H), 6.71-6.72 (d, 1H), 3.88 (s, 3H), 3.14-3.17 (m, 2H), 2.49-2.56 (m, 2H)

LCMS (ESI): m/z 291[M+H], 293[M+3H]

### Example 6: Preparation of 8-Bromo-1-ethylidene-2,2-difluoro-6-methoxy-1,2,3,4-tetrahydronaphthalene

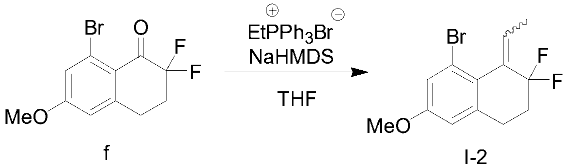

Ethyltriphenylphosphonium bromide (18.2 g, 1.5 eq) and tetrahydrofuran (76 mL, 8 V) were added to a reaction flask. In a nitrogen atmosphere, the mixture was cooled in an ice-water bath. With the temperature controlled at 0-10 °C, NaHMDS (22.0 mL, 1.35 eq) was added dropwise. After the addition, the mixture was left to react at 0-10 °C for 15 min. With the temperature controlled at 0-10 °C, a solution of f (9.5 g, 1.0 eq) in tetrahydrofuran (47.5 mL, 5 V) was added dropwise to the reaction flask. After the addition, the mixture was heated to 60 °C and left to react for 2 h. HPLC analysis showed that the reaction was complete. The reaction mixture was slowly cooled. With the temperature controlled at 0-10 °C, saturated ammonium chloride (47.5 mL, 5 V) and purified water (28.5 mL, 3 V) were added dropwise to the reaction mixture. The mixture was stirred, and layers formed. The aqueous phase was extracted once with ethyl acetate (47.5 mL, 5 V), and the organic phases were combined, washed once with saturated sodium chloride (28.5 mL, 3 V), dried over anhydrous sodium sulfate, and filtered. The filtrate was concentrated to dryness and then slurried with n-heptane (95 mL, 10 V) at room temperature (20-30 °C) for 1 h. After filtration, the filtrate was concentrated to dryness to give a crude product, and the crude product was purified by column chromatography (PE/EA = 100/1) to give a product (8.78 g, yield: 88.7%, purity: 90.78%). ¹HNMR (400 MHz, CDCl3): δ 7.04-7.05 (d., 1H), 6.64-6.67 (m, 2H), 3.78 (s, 3H), 2.67-2.71 (m, 2H), 2.11-2.19 (m, 5H)

### Example 7: Preparation of 8-Bromo-1-ethyl-2-fluoro-6-methoxynaphthalene

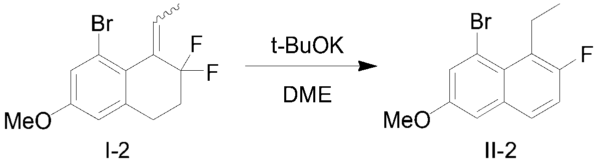

**I-2** (10.3 g, 1.0 eq) and dimethoxyethane (103 mL, 10 V) were added to a reaction flask. In a nitrogen atmosphere, with the temperature controlled at 0-20 °C, potassium tert-butoxide (5.72 g, 1.5 eq) was added. After the addition, the mixture was heated to 80 °C and left to react for 2 h. HPLC analysis showed that the reaction was complete. The reaction mixture was cooled to room temperature and filtered through diatomaceous earth. The filtrate was concentrated to dryness, and the residue was dissolved in DCM (103 mL, 10 V). The solution was washed once with saturated ammonium chloride (51.5 mL, 5 V), washed once with purified water (51.5 mL, 5 V), washed once with saturated sodium chloride (51.5 mL, 5 V), dried over anhydrous sodium sulfate, filtered, and concentrated to dryness to give a crude product (9.0 g, yield: 93.5%, purity: 79.8%). The crude product was directly used in the next step.

¹HNMR (400 MHz, CDCl3): δ 7.58-7.59 (d.,1H), 7.54-7.56 (m,1H), 7.21-7.25 (m, 1H), 7.09-7.10 (d,1H), 3.88 (s,3H), 3.49-3.55 (m,2H), 1.32-1.36 (t,3H)

### Example 8: Preparation of 4-Bromo-5-ethyl-6-fluoronaphthalen-2-ol

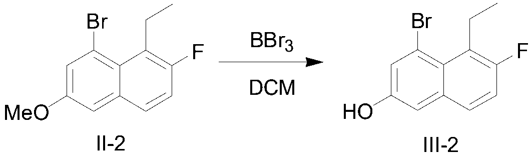

**II-2** (8.9 g, 1.0 eq) and dichloromethane (89 mL, 10 V) were added to a reaction flask. In a nitrogen atmosphere, the mixture was cooled in an ice-water bath. With the temperature controlled at 0-10 °C, boron tribromide (10.2 g, 1.3 eq) was slowly added dropwise. After the addition, the mixture was naturally warmed to room temperature and left to react for 1 h. HPLC analysis showed that the reaction was complete. With the temperature controlled at 0-10 °C, purified water (44.5 mL, 5 V) was added dropwise to quench the reaction, and layers formed. The organic phase was washed once with saturated sodium bicarbonate (71.2 mL, 8 V), dried over anhydrous sodium sulfate, filtered, and concentrated to dryness to give a product (8.4 g, yield: 99.3%, purity: 83.1%). The crude product was directly used in the next step.

¹HNMR (400 MHz, CDCl3): δ 7.54-7.55 (d,1H), 7.49-7.53 (dd, 1H), 7.20-7.24 (t, 1H), 7.12-7.13 (d, 1H), 4.97 (s, 1H), 3.48-3.55 (m, 2H), 1.32-1.36 (t, 3H)

### Example 9: Preparation of 8-Bromo-1-ethyl-2-fluoro-6-methoxymethoxynaphthalene

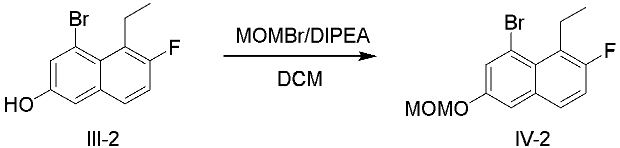

**III-2** (8.4 g, 1.0 eq), dichloromethane (84 mL, 10 V), and DIPEA (8.88 g, 2.2 eq) were added to a reaction flask. In a nitrogen atmosphere, the mixture was cooled in an ice-water bath. With the temperature controlled at 0-10 °C, bromomethyl methyl ether (6.63 g, 1.7 eq) was added dropwise. After the addition, the mixture was left to react at room temperature (20-30 °C) for 2 h. HPLC analysis showed that the reaction was complete. Purified water (84 mL, 10 V) was added. The mixture was stirred, and layers formed. The organic phase was dried over anhydrous sodium sulfate and filtered, and the filtrate was concentrated to dryness. The residue was purified by column chromatography (PE/EA = 50/1) to give a product (7.05 g, yield: 72.1%, purity: 91.02%).

¹HNMR (400 MHz, CDCl3): δ 7.65-7.66 (d,1H), 7.55-7.59 (dd, 1H), 7.35-7.36 (d, 1H), 7.23-7.26 (m, 1H), 5.25 (s, 2H), 3.49-3.56 (m, 5H), 1.32-1.36 (t, 3H)

### Example 10: Preparation of 2-(8-Ethyl-7-fluoro-3-(methoxymethoxy)naphthalen-1-yl)-4,4,5,5-tetramethyl-1,3,2-dioxaborolane

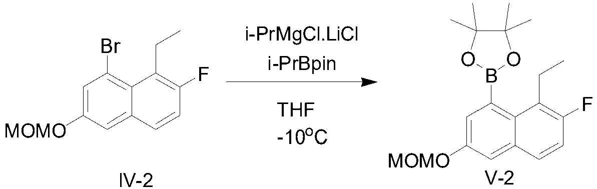

**IV-2** (5.0 g, 1.0 eq) and anhydrous tetrahydrofuran (50 mL, 10 V) were added to a reaction flask. The system was purged with argon. In an argon atmosphere, the reaction mixture was cooled to -10 °C. With the temperature controlled at -10 °C to -5 °C, a solution of isopropylmagnesium chloride-lithium chloride in tetrahydrofuran (15.96 mL, 1.3 eq) was added dropwise. After the addition, the mixture was left to react at -10 °C for 1 h. With the temperature controlled at -10 °C to -5 °C, isopropyl pinacol borate (5.94 g, 2.0 eq) was added dropwise. After the addition, the mixture was left to react at -10 °C for 2 h. HPLC in-process control showed that the reaction was complete. A saturated ammonium chloride solution (25 mL, 5 V) was added dropwise to quench the reaction, and purified water (50 mL, 10 V) and ethyl acetate (25 mL, 5 V) were then added. The mixture was stirred, and layers formed. The aqueous phase was extracted once with ethyl acetate (15 mL, 3 V), and the organic phases were combined, washed once with saturated sodium chloride (25 mL, 5 V), dried over anhydrous sodium sulfate, and filtered. The filtrate was concentrated to dryness to give a crude product. Methanol (10 mL, 2 V) was added to the crude product, and the mixture was heated to 60 °C and stirred until the crude product was completely dissolved. The solution was slowly cooled and stirred for crystallization. After filtration, the filter cake was dried to give a product (4.78 g, yield: 83.1%, purity: 98.68%).

¹HNMR (400 MHz, CDCl3): δ 7.55-7.58 (m,1H), 7.36-7.40 (dd, 2H), 7.18-7.22 (t, 1H), 5.27 (s, 2H), 3.50 (s, 3H), 3.11-3.14 (m, 2H), 1.44 (s, 12H), 1.25-1.29 (t, 3H)

## Claims

1. A method for preparing a compound represented by formula II, comprising a step of preparing the compound represented by formula II from a compound represented by formula I: 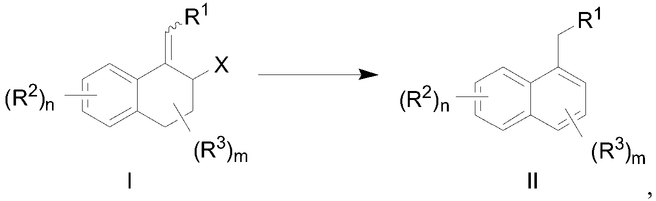 wherein:
X is a halogen;
R¹ is hydrogen or alkyl optionally substituted with one or more substituents Q;
R² are identical or different and are each independently selected from the group consisting of alkyl, alkoxy, halogen, hydroxy, sulfhydryl, oxo, -NRᵢRⱼ, cycloalkyl, heterocyclyl, aryl, and heteroaryl, wherein the alkyl, alkoxy, cycloalkyl, heterocyclyl, aryl, and heteroaryl are optionally substituted with one or more substituents Q;
R³ are identical or different and are each independently selected from the group consisting of alkyl, alkoxy, halogen, hydroxy, sulfhydryl, oxo, -NRᵢRⱼ, cycloalkyl, heterocyclyl, aryl, and heteroaryl, wherein the alkyl, alkoxy, cycloalkyl, heterocyclyl, aryl, and heteroaryl are optionally substituted with one or more substituents Q;
the substituents Q are each independently selected from the group consisting of C₁-C₆ alkyl, halogen, hydroxy, sulfhydryl, -NRᵢRⱼ, oxo, thio, -C(O)Rₖ, -C(O)ORₖ, -S(O)Rₖ,-S(O)ORₖ, -S(O)(O)Rₖ, -S(O)(O)ORₖ, -C(S)Rₖ, nitro, cyano, C₁-C₆ alkoxy, C₁-C₆ alkylthioether group, C₂-C₆ alkenyl, C₂-C₆ alkynyl, 3- to 10-membered cycloalkyl, 3- to 10-membered heterocyclyl, 6- to 10-membered aryl, 5- to 10-membered heteroaryl, 8- to 12-membered fused cycloaryl, and 5- to 12-membered fused heteroaryl;
Rᵢ and Rⱼ are each independently selected from the group consisting of hydrogen, hydroxy, C₁-C₆ alkyl, and C₁-C₆ alkoxy; Rₖ is independently selected from the group consisting of hydrogen, C₁-C₆ alkyl, C₁-C₆ haloalkyl, C₁-C₆ alkoxy, hydroxy, and -NRᵢRⱼ, wherein the alkyl and alkoxy are optionally substituted with one or more substituents selected from the group consisting of C₁-C₆ alkyl, halogen, hydroxy, sulfhydryl, -NRᵢRⱼ, oxo, thio, carboxyl, nitro, cyano, C₁-C₆ alkoxy, C₁-C₆ alkylthioether group, C₂-C₆ alkenyl, C₂-C₆ alkynyl, 3- to 10-membered cycloalkyl, 3- to 10-membered heterocyclyl, 6- to 10-membered aryl, and 5- to 10-membered heteroaryl;
n is 0, 1, 2, 3, or 4;
m is 0, 1 or 2, or 3.

2. The method according to claim 1, wherein R² are each independently selected from the group consisting of halogen and C₁-C₆ alkoxy, and n is 2.

3. The method according to claim 1 or 2, comprising a step of preparing a compound represented by formula II-1 from a compound represented by formula I-1: 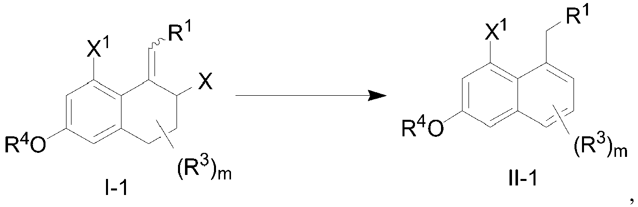 wherein:
X¹ is a halogen;
R⁴ is alkyl optionally substituted with one or more substituents Q;
R¹, R³, X, Q, and m are as defined in claim 1.

4. A method for preparing a compound represented by formula III-1, comprising the method according to claim 3, and further comprising a step of hydroxylating the compound represented by formula II-1 to give the compound represented by formula III-1: 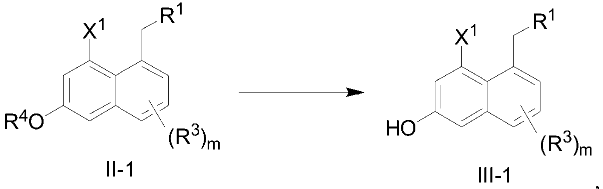 wherein R¹, R³, R⁴, X¹, and m are as defined in claim 3.

5. A method for preparing a compound represented by formula IV-1, comprising the method according to claim 4, and further comprising a step of preparing the compound represented by formula IV-1 from the compound represented by formula III-1: 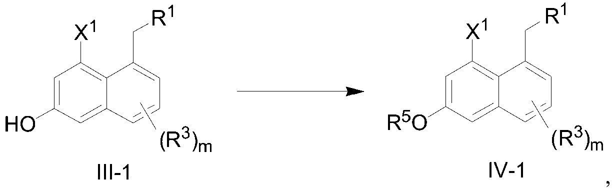
wherein R¹, R³, X¹, and m are as defined in claim 1;
R⁵ is a hydroxy protecting group preferably selected from the group consisting of benzyl, benzoyl, tert-butyryl, trimethylsilyl, tert-butyldiphenylsilyl, and methoxymethyl.

6. A method for preparing a compound represented by formula IV-1, comprising the method according to claim 5, and further comprising a step of preparing the compound represented by formula V-1 from the compound represented by formula IV-1: 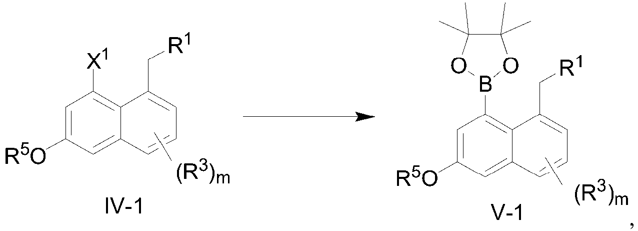 wherein R¹, R³, R⁵, X¹, and m are as defined in claim 5.

7. A compound represented by formula f:

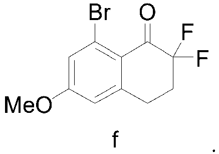

8. A compound represented by formula I-2:

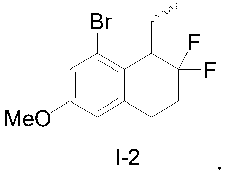

9. A compound represented by formula II-2:

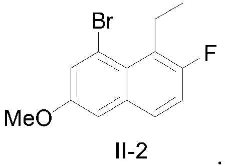

10. A compound represented by formula III-2:

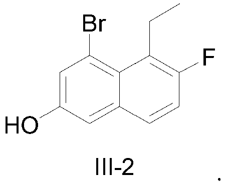

11. A compound represented by formula IV-2:

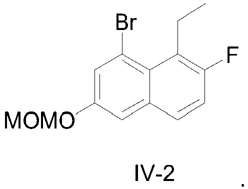

12. A method for preparing a compound represented by formula V-2, comprising the following steps:

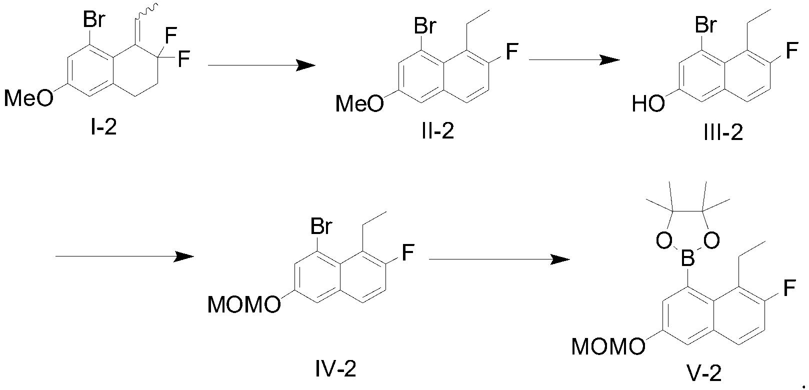

13. A method for preparing a KRAS G12D compound, comprising the method according to claim 1.

14. Use of a compound represented by formula II in the manufacture of a KRAS G12D compound, wherein the compound represented by formula II is prepared by the method according to claim 1.

15. A method for preparing a compound represented by formula A, comprising the method according to any one of claims 1-12:
